# EUROPÄISCHE PATENTSCHRIFT

(11) **EP 0 806 952 B1**
(45) Veröffentlichungstag und Bekanntmachung des Hinweises auf die Patenterteilung: **02.04.2003**
(21) Anmeldenummer: 95919986.0
(22) Anmeldetag: 02.02.1995
(51) Int. Cl.: A61K 31/57, A61K 31/575

(54) **VERWENDUNG VON KOMPETITIVEN PROGESTERONANTAGONISTEN FÜR DIE HERSTELLUNG VON ARZNEIMITTELN ZUR BEHANDLUNG VON DYSFUNKTIONELLEN UTERINEN BLUTUNGEN**
USE OF COMPETITIVE PROGESTERONE ANTAGONISTS FOR THE PREPARATION OF A MEDICAMENT FOR THE TREATMENT OF DYSFUNCTIONAL UTERINE BLEEDING
UTILISATION D' ANTAGONISTES DE LA PROGESTERONE COMPETITIFS POUR LA PREPARATION D'UN MEDICAMENT POUR LE TRAITEMENT D'HEMORRAGIES UTERINES DYSFONCTIONNELLES

(43) Veröffentlichungstag der Anmeldung: 19.11.1997
(73) Patentinhaber: SCHERING AKTIENGESELLSCHAFT, 13353 Berlin (DE)
(72) Erfinder: CHWALISZ, Kristof, 13503 Berlin (DE); STÖCKEMANN, Klaus, 12161 Berlin (DE)
(86) Internationale Anmeldenummer: EP9500394
(87) Internationale Veröffentlichungsnummer: WO96023503

(56) Entgegenhaltungen:
- WO-A-93/21927
- DATABASE WPI Section Ch, Week 8823 Derwent Publications Ltd., London, GB; Class B01, AN 88-159461 ANONYMOUS 'Use of progesterone antagonists e.g. miferpristone or lilopristone - for treating glaucoma, cushing's syndrome, as immunostimulant, antihypertensive, chronic corticosteroid adjuvant' & RESEARCH DISCLOSURE, Bd. 289, Nr. 076, 10.Mai 1988 EMSWORTH, GB,
- INT. J. FERTIL., 1993, 38/5 (261-269), USA, AWWAD J.T. ET AL 'Abnormal uterine bleeding in the perimenopause'

## Beschreibung

Die vorliegende Erfindung betrifft die Verwendung mindestens einer Verbindung mit progesteronantagonistischer (PA) Wirkung zur Herstellung von Arzneimitteln für die Behandlung von dysfunktionellen uterinen Blutungen, aus der gruppe bestehend aus Metrorrhagien, Menorrhagien und Hypermenorrhea.
Dysfunktionelle uterine Blutungen (dysfunctional or abnormal uterine bleeding, Metrorrhagien und Menorrhagien, Hypermenorrhea) sind pathologische Blutungen, die nicht auf organische Veränderungen im Uterus (wie z.B. Endometrialkarzinom, Myome, Polypen etc.), systemische Koagulationsstörungen oder auf eine pathologische Schwangerschaft (z.B. ektopische Schwangerschaft, drohender Abort) zurückzuführen sind (American College of Obstetricians and Gynccologists, 1982).
Der durchschnittliche Blutverlust während einer normalen Menstruation beträgt etwa 30 ml, wobei die Periode durchschnittlich 5 Tage dauert. Übersteigt der menstruale Blutverlust 80 ml, so wird er als pathologisch eingestuft (Zahradnik HP, (1992) Menstruation. In Käser O et al. (editors) Gynäkologie und Geburtshilfe. Band 1/2. Georg Thieme Verlag Stuttgart, New York: 7.31-7.51).
Metrorrhagien werden als schmerzfreie oder schmerzhafte Blutungen definiert, deren menstruale und zyklische Zuordnung unmöglich ist. Bei einer Dauer von über 7 Tagen liegt der Blutverlust oft über 80 ml.
Menorrhagie ist eine schmerzfreie oder schmerzhafte Menstruation, normalerweise alle 27-28 Tage, die bei einer Dauer von über 7 Tagen meistens mit einem vermehrten Blutverlust von über 80 ml einhergeht.
Hypermenorrhea wird definiert als eine schmerzfreie oder schmerzhafte Menstruation, normalerweise alle 27-28 Tage, für 4-5 Tage bei einem vermehrtem Blutverlust von über 80 ml.
RD-289076 erwähnt die Verwendung von RU 486 und Lilopristine (ZK 98.734) zur Behandlung von idiopathischen Uterinen Blutungen.
Dysfunktionelle uterine Blutungen (hauptsächlich Metrorrhagien und Menorrhagien) sind typisch für die Adoleszenz und für die Zeit des Klimakteriums, in denen es gehäuft zu Follikelreifungsstörungen, Anovulation, Gelbkörper- und Follikelpersistenz kommt. Die Inzidenz von dysfunktionellen uterinen Blutungen ist hoch und stellt einen der häufigsten Gründe für eine gynäkologische Beratung für Frauen im reproduktionsfähigen Alter dar. Die Konsultationsrate wegen dysfunktionellen ulerinen Blutungen beträgt 33% im reproduktionsfähigen Alter und 69% in der Peri- und Postmenopause (Mencaglia L., Perino A., Hamon J. (1987) Hysterescopy in perimenopausal and postmenopausal women with abnormal uterine bleeding. J. Reprod. Med. 32:577).

Die Vielfalt der angewandten Therapien bei dysfunktionellen uterinen Blutungen ist ein indirekter Hinweis dafür, daß die Pathogenese dieser Erkrankung noch nicht geklärt ist und eine wirksame Therapie bisher nicht existiert. Derzeit werden dysfunktionelle uterine Blutungen mil Gestagenen (z.B. 10 mg Medroxyprogesteronacetat täglich, 0.7-1.0 mg Norethindroneacetat täglich, jeweils für 10-14 Tage), hochdosierten Östrogen/Gestagen-Kombinationen über einen Zeitraum von 10-14 Tagen, aber auch mit nichtsteroidalen Zyklooxygegenasehemmern (z.B. Mefenaminsäure, Naproxen, Ibuprofen) und LHRH-Agonisten behandelt (Cowan BD (1992) Dysfunctional uterine bleeding: Clues to efficacious Approaches. In Alexander NJ, d'Arangues C (editors), Steroid hormones and uterine bleeding. AAAS Press, 1922: 9-17).

Die Therapie mit einer hochdosierten Östrogen/Gestagen-Kombination ist mit den bekannten kardiovaskulären Risiken verbunden (überwiegend thromboembolische Erkrankungen). Ist die medikamentöse Therapie von dysfunktionellen uterinen Blutungen nicht erfolgreich, werden chirurgische Methoden (uterine Kürretage, Hysterektomie) empfohlen. Die dysfunktionellen uterinen Blutungen stellen nach den Uterusmyomen die zweithäufigste Indikation für diese Operation dar (Lee NC, Dicker RC, Rubin GL, Ory HW (1984) Confirmation of the prcoperative diagnosis for hysterectomie. Am. J. Obstet. Gynecol., 150: 283). Es ist bemerkenswert, daß die Zahl von Hysterektomien wegen dysfunktionellen uterinen Blutungen. insbesondere bei prämenopausalen Frauen, in letzter Zeit nicht reduziert werden konnte, obwohl eine Vielfalt von medikamentösen Therapiemöglichkeiten zur Verfügung steht (Lumsden MA, (1990) Menorrhagia- the cost and scope of treatment. In Shaw RW. (editor) Dysfunctional uterine bleeding. The Parthenon Publishing Group: 85-96). Die Hysterektomie beinhaltet nicht zu unterschätzende Risiken. Die Mortalitätsrate nach einer Hysterektomie beträgt 6 per 100 000 (Wingo et al., 1985).

Der Erfindung liegt daher die Aufgabe zugrunde, ein Arzneimittel für die angegebene Indikation bereitzustellen, welches dysfunktionelle Blutungen zum Stillstand bringt und die unerwünschten Wirkungen von hochdosierten Östrogen/Gestagen-Präparaten nicht oder nur in geringem Maße aufweist.

Einen derartigen neuen medikamentösen Ansatz stellt die Verwendung kompetitiver Progesteronantagonisten (Antigestagene) zur Herstellung eines Arzneimittels für die angegebene Indikation dar.

Antigestagene, wenn sie in der Lutealphase gegeben werden, sind in der Lage, eine menstruationsähnliche Blutung zu induzieren (Nieman LK, Healy DL, Spitz IM, Nisula BC, Merriam GR, Bardin CW, Loriaux DL, Chrousos GP (1985) Use of single doses of the antiprogesterone steroid RU 486 for induction of menstruation in normal women. In Baulieu EE: Segal SJ (cditors): The antiprogestin steroid RU 486 and human fertility control. Plenum Press, New York and London: 279-285). Experimente an Primaten zeigen, daß die antigestageninduziertc Blutung zu einer vollständigen Eliminierung des Endometriumsgewebes führt (Chilik CF, Hsiu JG, Acosta AA, van Uem JFHM, Hodgen GD (1986) RU 486-induced Menses in cynomolgus monkeys: uniformity of endometrial sloughing. Fertil Steril 45:708). Der genaue Mechanismus der Blutungsinduktion durch den Progesteronabfall in einem normalen Zyklus bzw. durch eine Antigestagenbehandlung ist noch nicht geklärt. Es wird angenommen, daß der Progesteronentzug zu einer Induktion von uterinen Prostaglandinen führt, die eine Blutung induzieren (Zahradnik HP, (1992), loc. cit.)
Es ist auch bekannt, daß das Progesteron die Synthese von Endothelin im Uterus hemmt und dessen enzymatischen Abbau stimuliert (Casey ML, MacDonald PC (1992) Modulation of endometrial blood flow: Regulation of endothelin-1 biosynthesis and degradation in human endometrium. In Alexander NJ, d'Arcangues C (editors), Steroid hormones and uerine bleeding. AAAS Press, 1992: 210-224). Das Endothelin gilt als die potenteste vasokonstriktorische endogene Substanz. Es wird angenommen, daß die vermehrte Endothelinfreisetzung am Ende eines normalen Zyklus infolge des Progesteronabfalls zur Konstriktion von Spiralarterien im Endometrium führt und dadurch eine Menstruationsblutung induziert (Casey und MacDonald (1992), loc. cit.).

Es wurde nunmehr gefunden, daß kompetitive Progesteronantagonisten überraschenderweise in der Lage sind, aufgrund einer Vasokonstriktion von endometrialen Arterien durch die Steigerung der Uteruskontraktionen und die dadurch bedingte Wirkung auf das Myometrium, bei dysfunktionellen uterinen Blutungen einen Blutungsstillstand herbeizuführen. Im Falle von dysfunktionellen uterinen Blutungen scheint nämlich die Aktivierung von Mechanismen, die zur Vasokonstriktion von endometrialen Arterien führen, aufgrund eines nicht vollständigen bzw. zu lang andauernden Progesteronabfalls nicht adäquat zu sein.

Unerwünschte Nebenwirkungen, wie sie bei der Behandlung mit hochdosierten Östrogen/Gestagen-Präparaten auftreten können, werden bei Verwendung von kompetitiven Progesteronantagonisten nicht beobachtet. Diese können als Substanzen ohne nenneswerte Nebenwirkungen angesehen werden.

Über die Behandlung von dysfunklionellen uterinen Blutungen mit Antigestagenen im normalen Zyklus ist bisher nichts bekannt. Vielmehr ist die Aufmerksamkeit auf die Anwendung von Gestagenen für diese Indikation gerichtet (Cowan 1992, loc. cit.). Daß sich das Auftreten von Durchbruchblutungen, die unter der Behandlung mit einem niedrig dosierten, Östrogen- und Gestagen-haltigen oralen Kontrazeptivum durch die ungenügende Zykluskontrolle aufgrund der niedrigen Dosierung zustande kommen, reduzieren läßt, ist in der internationalen Patentanmeldung WO-A-93/17686 beschrieben. Die Verwendung progesteronantagonistisch wirksamer Verbindungen allein oder in Kombination mit antiöstrogen wirksamen Verbindungen zur Herstellung von Arzneimitteln zur Geburtseinleitung, zum Schwangerschaftsabbruch sowie zur Behandlung gynäkologischer Störungen (Dysmenorrhea, Endometriose) geht bereits aus der EP-A-03110541 hervor.

Die erfindungsgemäß hergestellten Arzneimittel sind für die Behandlung aller Formen von dysfuntionellen uterinen Blutungen wie Menorrhagien, Metrorrhagien und Hypermenorrhoe geeignet.

Als kompetitive PA kommen alle Verbindungen in Frage, die selbst oder deren Metabolite die Wirkung des Progesterons an dessen Rezeptor blockieren; beispielweise folgende Steroide:
11β-((4-N,N-Dimethylamino)-phenyl)-17β-hydroxy-17α-propinyl-4,9 (10)-estradien-3-on (RU-38486),
11 ß-((4-N,N-Dimethylamino)-phenyl)-17β-hydroxy-18-methyl-17α-propinyl-4,9 (10)-estradien-3-on und
11β-((4-N,N-Dimethylamino)-phenyl)-17aβ-hydroxy-17aα-propinyl-D-homo-4,9 (10),16-estratien-3-on (alle EP-A-0 057 115), ferner
11β-p-Methoxyphenyl-17β-hydroxy-17α-ethinyl-4,9 (10)-estradien-3-on (Steroids 37 (1981), 361-382),
11β-(4-Acetylphenyl)-17β-hydroxy-17α-(prop-1-inyl)-4,9 (10)-estradien-3-on (EP-A 0 190 759),
sowie die in der EP-A 0 277 676 beschriebenen 11β-Aryl-14β-estradiene und -triene, die 19,11β-überbrückten Steroide, die Gegenstand der EP-A-0 283 428 sind, die aus der EP-A-0 289 073 hervorgehenden 11β-Aryl-6-alkyl (bzw. 6-Alkenyl oder 6-alkinyl)-estradiene und - pregnadiene und die aus der EP-A-0 321 010 bekannten 11β-Aryl-7-methyl (bzw. 7-ethyl)-estradiene sowie die 10β-H-Steroide der EP-A-0 404 283, beispielsweise (Z)-11β-[4-(Dimethylamino)phenyl]-17β-hydroxy-17α-(3-hydroxy-1-propenyl)-estr-4-en-3-on.

Weiterhin seien als typische Vertreter erfindungsgemäß zu verwendender, kompetitiver Progesteronantagonisten beispielsweise genannt:
11β-(4-Dimethylaminophenyl)-17α-hydroxy-17β-(3-hydroxy-propyl)-13α-methyl-4,9-gonadicn-3-on (EP-A-0 190 759);
11β,19-(4-Acetylphenyl)-17β-hydroxy-17α-(3-hydroxyprop-1-(Z)-enyl)-4,9(10)-estradien-3-on (EP-A-0 190 759);
11β,19-(4-(Cyanphenyl)-17β-hydroxy-17α-(3-hydroxyprop-1-(Z)-enyl)-4-androsten-3-on und
11β,19-(4-(3-Pyridinyl)-o-phenylen)-17β-hydroxy-17α-(3-hydroxyprop-1-(Z)-enyl)-4-androsten-3-on (beide WO-A-93/23020).

Zur Therapie von dysfunktionellen uterinen Blutungen mit PA ist erfindungsgemäß im allgemeinen eine kurzzeitige Behandlung (täglich an 1 bis maximal 10 Tagen) mit einer täglichen Dosis von 1 bis 600 mg des kompetitiven Progesteronantagonisten pro Tag ausreichend. Vorzugsweise wird erfindungsgemäß täglich eine Menge von 5 bis 400 mg appliziert. Insbesondere bevorzugt ist die tägliche Applikation von 50 bis 400 mg 11β-(4-Dimethylaminophenyl)-17α-hydroxy-17β-(3-hydroxy-propyl)-13α-methyl-4,9-gonadien-3-on (Onapriston) oder von 50 bis 400 mg (Z)-11β-[4-(Dimethylamino)phenyl]-17β-hydroxy-17α-(3-hydroxy-1-propenyl)-estr-4-en-3-on.
Es kann bereits die einmalige Gabe eines kompetitiven Progesteronantagonisten innerhalb des angegebenen Dosierungsbereiches genügen, um die Blutungen zum Stillstand zu bringen, vor allem wenn eine Dosierung innerhalb des insbesondere bevorzugten Dosisbereichs von Onapriston oder (Z)-11β-[4-(Dimethylamino)phenyl]-17β-hydroxy-17α-(3-hydroxy-1-propenyl)-estr-4-en-3-on gewählt wird. Ansonsten wird die Behandlung bis zum Stillstand der Blutungen fortgeführt, der meist innerhalb von spätestens 5 Tagen eintritt. Erfindungsgemäß ist sowohl die akute Behandlung auftretender dysfunktioneller uteriner Blutungen als auch die prophylaktische Vorbeugung solcher Blutungen möglich.

Der kompetitive Progesteronantagonist kann für die erfindungsgemäße Verwendung z.B. lokal, enteral oder parenteral appliziert werden. Für die bevorzugte enterale Applikation kommen insbesondere Tabletten, Dragees, Kapseln, Pillen, Suspensionen oder Lösungern in Frage, die in üblicher Weise mit den in der Galenik üblichen Zusätzen und Trägersubstanzen hergestellt werden können. Für die lokale oder topische Anwendung kommen beispielweise Vaginalzäpfchen in Frage.

Das nachfolgende Beispiel zeigt exemplarisch die Formulierung eines kompetitiven Progesteronantagonisten für die erfindungsgemäße Verwendung. Andere Progesteronantagonisten können ganz analog formuliert werden, wobei in der Formulierung jede vorstehend als geeignet beschriebene Menge enthalten sein kann.

### Formulierungsbeispiel:

| | |
|---|---|
| 100,0 mg | 11β-[(4-N,N-Dimethylamino)-phenyl]-17α-hydroxy-17b-(3-hydroxypropyl)-13α-methyl-4,9-gonadien-3-on |
| 140.5 mg | Laktose |
| 69,5 mg | Maisstärke |
| 2,5 mg | Polyvinylpyrrolidon |
| 2,0 mg | Aerosil |
| 0.5 mg | Magnesiumstearat |
| 315,0 mg | Gesamtgewicht der Tablette, die in üblicher Weise auf einer |
| ===== | Tablettenpresse hergestellt wird. |

### Beispiel 1

### Akute Behandlung von Metrorrhagien durch einmalige Onapristongabe

Frauen mit dysfunktionellen Metrrorhagien werden einmalig mit 200-400 mg Onapriston oral behandelt. Bevorzugt ist eine Behandlung in der Lutealphase des Zyklus. Die Behandlung führt zum Blutungsstillstand innerhalb von 2-4 Tagen.

### Beispiel 2

### Akute Behandlung von Menorrhagien und Hypermenorrhoea durch einmalige Onapristongabe

Frauen mit Menorrhagien und Hypermenorrhoea werden am 28. Tag des Zyklus bzw. am ersten Tage der Blutung einmalig mit 200-400 mg Onapriston oral behandelt. Die Behandlung induziert eine menstruationsähnliche Blutung normaler Intensität (ca. 30 ml) und Dauer (ca. 5 Tage).

### Beispiel 3

### Prophylaktische Behandlung von Metrorrhagien durch einmalige Onapristongabe

Frauen mit dysfunktionellen Metrorrhagien werden einmalig mit 200-400 mg Onapriston alle 28 Tage oral behandelt. Die Behandlung induziert eine menstruationsähnliche Blutung normaler Intensität (ca. 30 ml) und Dauer (ca. 5 Tage) und verhindert das Auftreten von Metrorrhagien. Die Behandlung wird über 3-6 Zyklen fortgestetzt.

### Beispiel 4

### Prophylaktische Behandlung von Menorrhagien und Hypermenorrhoea durch einmalige Onapristongabe

Frauen mit Menorrhagien und Hypermenorrhoea werden alle 28 Tage mit 200-400 mg Onapriston oral behandelt. Die Behandlung induziert eine menstruationsähnliche Blutung normaler Intensität (ca. 30 ml) und Dauer (ca. 5 Tage) und verhindert das Auftreten von Menorrhagien und Hypermenorrhoea. Die Behandlung wird über 3-6 Zyklen fortgesetzt.

### Beispiel 5

### Akute Behandlung von Metrorrhagien durch mehrmalige Verabreichung von Onapriston

Frauen mit dysfunktionellen Metrorrhagien werden mit 200-400 mg/Tag Onapriston oral bis zum Blutungsstillstand behandelt, max. über 10 Tage. Der Anfang der Behandlung soll vorzugsweise in der Lutealphase des Zyklus liegen.

### Beispiel 6

### Akute Behandlung von Menorrhagien und Hypermenorrhoea durch mehrmalige Verabreichung von Onapriston

Frauen mit Menorrhagien und Hypermenorrhoea werden am 28. Tage des Zyklus bzw. am ersten Tage der Blutung mit 200-400 mg/Tag Onapriston oral bis zum Blutungsstillstand, maximal über 10 Tage, behandelt. Die Behandlung induziert eine menstruationsähnliche Blutung normaler Intensität (ca. 30 ml) und Dauer (ca. 5 Tage) und verhindert dadurch vermehrten Blutverlust.

### Beispiel 7

### Prophylaktische Behandlung von Metrorrhagien durch mehrmalige Verabreichung von Onapriston

Frauen mit dysfunktionellen Metrorrhagien werden max. 10 Tage lang mit 200-400 mg/Tag Onapriston oral alle 28 Tage behandelt. Die Behandlung induziert eine menstruationsähnliche Blutung normaler Intensität (ca. 30 ml) und Dauer (ca. 5 Tage) und verhindert das Auftreten von Metrorrhagien. Die Behandlung wird über 3-6 Zyklen fortgesetzt.

### Beispiel 8

### Prophylaktische Behandlung von Menorrhagien und Hypermenorrhoea durch mehrmalige Verabreichung von Onapriston

Frauen mit Menorrhagien und Hypermenorrhoea werden alle 28 Tage max. 10 Tage lang mit 200-400 mg/Tag Onapriston oral behandelt. Die Behandlung induziert eine menstruationsähnliche Blutung normaler Intensität (ca. 30 ml) und Dauer (ca. 5 Tage) und verhindert das Auftreten von Menorrhagien und Hypermenorrhoea. Die Behandlung wird über 3-6 Zyklen fortgesetzt.

Bei der erfindungsgemäßen Behandlung dysfunktioneller uteriner Blutungen unter Verwendung eines kompetitiven Progesteronantagonisten wird also sowohl die ansonsten beobachtete Blutungsdauer verkürzt als auch die Intensität, d. h. der Blutverlust drastisch verringert.

## Patentansprüche

1. Verwendung von kompetitiven Progesteronantagonisten (PA) zur Herstellung von Arzneimitteln zur Behandlung dysfunktioneller uteriner Blutungen aus der Gruppe bestehend aus Metrorrhagien, Menorrhagien und Hypermenorrhea.

2. Verwendung von
11β-((4-N,N-Dimethylamino)-phenyl)-17β-hydroxy-17α-propinyl-4,9(10)-estradien-3-on (RU-38486),
11β-((4-N,N-Dimethylamino)-phenyl)-17β-hydroxy-18-methyl-17α-propinyl-4,9(10)-estradien-3-on,
11β-((4-N,N-Dimethylamino)-phenyl)-17aß-hydroxy-17aα-propinyl-D-homo-4,9 (10),16-estratien-3-on,
11β-p-Methoxyphenyl-17β-hydroxy-17α-ethinyl-4,9(10)-estradien-3-on,
11β-(4-Acetylphenyl)-17β-hydroxy-17α-(prop-1-inyl)-4,9 (10)-estradien-3-on,
11β-(4-Dimethylaminophenyl)-17α-hydroxy-17ß-(3-hydroxy-propyl)-13α-methyl-4,9-gonadien-3-on,
(Z)-11β-[4-(Dimethylamino)phenyl]-17β-hydroxy-17α-(3-hydroxy-1-propenyl)-estr-4-en-3-on5
11β,19-(4-Acetylphenyl)-17β-hydroxy-17α-(3-hydroxyprop-1-(Z)-enyl)-4,9(10)-estradien-3-on,
11β,19-(4-(Cyanphenyl)-17β-hydroxy-17α-(3-hydroxyprop-1-(Z)-enyl)-4-androsten-3-on oder
11β,19-(4-(3-Pyridinyl)-o-phenylen)-17β-hydroxy-17α-(3-hydroxyprop-1-(Z)-enyl)-4-androsten-3-on
als kompetitiver Progesteronantagonist nach Anspruch 1.

3. Verwendung von 1 bis 600 mg eines PA in einer täglichen Dosierungseinheit zur Herstellung eines Arzneimittels nach Anspruch 1.

4. Verwendung von 50 bis 400 mg eines PA nach Anspruch 3.

5. Verwendung von 50 bis 400 mg 11β-(4-Dimethylaminophenyl)-17α-hydroxy-17β-(3-hydroxy-propyl)-13α-methyl-4,9-gonadien-3-on oder
50 bis 400 mg (Z)-11β-[4-(Dimethylamino)phenyl]-17β-hydroxy-17α-(3-hydroxy-1-propenyl)-estr-4-en-3-on nach Anspruch 3.

## Claims

1. The use of competitive progesterone antagonists (PA) for the production of pharmaceutical agents for the treatment of dysfunctional uterine bleeding in the form of metrorrhagia, menorrhagia or hypermenorrhea.

2. The use as a competitive progesterone antagonist according to claim 1 of
11β-((4-N,N-Dimethylamino)-phenyl-17β-hydroxy-17α-propynyl-4,9(10)-estradien-3-one-(RU-38486),
11β-((4-N,N-dimethylamino)-phenyl)-17β-hydroxy-18-methyl-17α-propynyl-4,9(10)-estradien-3-one,
11β-((4-N,N-dimethylamino)-phenyl)-17aβ-hydroxy-17aα-propynyl-D-homo-4,9(10),16-estradien-3-one,
11β-p-methoxyphenyl-17β-hydroxy-17α-ethynyl-4,9(10)-estradien-3-one,
11β-(4-acetylphenyl)-17β-hydroxy-17α-(prop-1-ynyl)-4,9(10)estradien-3-one,
11β-(4-dimethylaminophenyl)-17α-hydroxy-17β-(3-hydroxy-propyl)-13α-methyl-4,9-gonadien-3-one,
(Z)-11β-[4-(dimethylamino)phenyl]-17β-hydroxy-17α-(3-hydroxy-1-propenyl)-estr-4-en-3-one-,
11β,19-(4-acetylphenyl)-17β-hydroxy-17α-(3-hydroxyprop-1-(Z)-enyl)-4,9(10)-estradien-3-one,
11β,19-(4-cyanophenyl)-17β-hydroxy-17α-(3-hydroxyprop-1-(Z)-enyl)-4-androsten-3-one or
11β,19-(4-(3-pyridinyl)-o-phenylene)-17β-hydroxy-17α-(3-hydroxyprop-1-(Z)-enyl)-4-androsten-3-one.

3. The use of 1 to 600 mg of a PA in a daily dosage unit for the production of a pharmaceutical agent according to claim 1.

4. The use of 50 to 400 mg of a PA according to claim 3.

5. The use according to claim 3 of 50 to 400 mg of 11β-(4-dimethylaminophenyl)-17α-hydroxy-17β-(3-hydroxypropyl)-13α-methyl-4,9-gonadien-3-one or 50 to 400 mg of (Z)-11β-[4-(dimethylamino)phenyl]-17β-hydroxy-17α-(3-hydroxy-1-propenyl)-estr-4-en-3-one.

## Revendications

1. Utilisation d'antagonistes compétitifs de la progestérone (AP) pour la production de médicaments destinés au traitement d'hémorragies utérines dysfonctionnelles parmi le groupe constitué de métrorragies, de ménorragies et d'hyperménorrhées.

2. Utilisation de
la 11β-((4-N,N-diméthylamino)phényl)-17β-hydroxy-17α-propynyl-4,9(10)-estradién-3-one (RU-38486),
la 11β-((4-N,N-diméthylamino)phényl)-17β-hydroxy-18-méthyl-17α-propynyl-4,9(10)-estradién-3-one,
la 11β-((4-N,N-diméthylamino)phényl)-17aβ-hydroxy-17aα-propynyl-D-homo-4,9(10),16-estradién-3-one,
la 11β-p-méthoxyphényl-17β-hydroxy-17α-éthinyl-4,9(10)-estradién-3-one,
la 11β-(4-acétylphényl)-17β-hydroxy-17α-(prop-1-ynyl)-4,9(10)-estradién-3-one,
la 11β-(4-diméthylaminophényl)-17α-hydroxy-17β-(3-hydroxypropyl)-13α-méthyl-4,9-gonadién-3-one,
la (Z)-11β-[4-(diméthylamino)phényl]-17β-hydroxy-17α-(3-hydroxy-1-propényl)-estr-4-én-3-one,
la 11β,19-(4-acétylphényl)-17β-hydroxy-17α-(3-(hydroxyprop-1-(Z)-ényl)-4,9(10)-estradién-3-one,
la 11β,19-(4-(cyanophényl)-17β-hydroxy-17α-(3-(hydroxyprop-1-(Z)-ényl)-4-androstén-3-one ou
la 11β,19-(4-(3-pyridinyl)-o-phénylène)-17β-hydroxy-17α-(3-(hydroxyprop-1-(Z)-ényl)-4-androstén-3-one,
en tant qu'antagoniste compétitif de la progestérone selon la revendication 1.

3. Utilisation de 1 à 600 mg d'un AP dans une dose unitaire journalière pour la production d'un médicament selon la revendication 1.

4. Utilisation de 50 à 400 mg d'un AP selon la revendication 3.

5. Utilisation de 50 à 400 mg de la 11β-(4-diméthylaminophényl)-17α-hydroxy-17β-(3-(hydroxypropyl)-13α-méthyl-4,9-gonadién-3-one ou de 50 à 400 mg de la (Z)-11β-[4-(diméthylamino)phényl]-17β-hydroxy-17α-(3-hydroxy-1-propényl)-estr-4-én-3-one selon la revendication 3.
